# EUROPEAN PATENT SPECIFICATION

(11) **EP 2 470 107 B1**
(45) Date of publication and mention of the grant of the patent: **14.08.2019**
(21) Application number: 10812398.5
(22) Date of filing: 19.08.2010
(51) Int. Cl.: A61M 25/02, A61B 46/23, A61B 46/20, A61B 46/00, A61B 46/27

(54) **A SURGICAL INSTRUMENT PLACEMENT TOOL FOR HOLDING TOOLS FOR ANGIOGRAPHIC PROCEDURES**
WERKZEUG ZUM PLATZIEREN EINES CHIRURGISCHEN INSTRUMENTS ZUM HALTEN VON WERKZEUGEN FÜR ANGIOGRAPHISCHE VERFAHREN
DISPOSITIF DE MISE EN PLACE D'UN INSTRUMENT CHIRURGICAL SERVANT À RETENIR DES OUTILS POUR DES PROCÉDURES ANGIOGRAPHIQUES

(30) Priority: 24.08.2009 SE 0950607
(43) Date of publication of application: 04.07.2012
(73) Proprietor: Mölnlycke Health Care AB, 402 52 Göteborg (SE)
(72) Inventor: HÖRER, Tal Martin, S-702 17 Örebro (SE)
(74) Representative: Kransell & Wennborg KB
(86) International application number: PCT/SE2010/050896
(87) International publication number: WO 2011/025439

(56) References cited:
- EP-A1- 0 487 074
- WO-A1-2009/049912
- US-A- 3 503 391
- US-A- 3 696 920
- US-A- 4 414 968
- US-A- 4 471 769
- US-A- 4 570 628
- US-A- 5 464 025
- US-A- 5 944 014
- US-A1- 2003 060 831
- US-B1- 6 436 085

## Description

### TECHNICAL FIELD

The present invention relates to a surgical instrument placement tool for holding tools for angiographic procedures in the fields of invasive radiology, vascular surgery and cardiology.

It also relates to a tool holder for holding tools for angiographic procedures in the fields of invasive radiology, vascular surgery and cardiology in case a surgical team operates at a table of extra length.

In addition, it relates to a pack of such tool holders.

### BACKGROUND ART

The use of catheters, spring guides wires, balloons and stents is increasing rapidly in the fields of invasive radiology, vascular surgery and cardiology. Millions of procedures are carried out every year throughout the world, as there is increasing tendency to treat vascular diseases with endovascular techniques. This is especially prominent in the field of vascular surgery, where there is an increasing tendency to choose endovascular methods for first line treatments.

The endovascular instruments are mainly introducers, catheters, spring guide wires, stents and balloons. There are many types of tools in different length and diameter from several centimetres to 3 meters in length. In the majority of procedures, at least 7-8 tools are used and in many others 10-20 tools and more.

To obtain safe access to blood vessels and other hollow organs, the Seldinger technique is a medical procedure often used. It is named after Dr. Sven-Ivar Seldinger, a Swedish radiologist, who introduced the procedure in 1953.

The desired vessel or cavity is punctured with a sharp hollow needle called a trocar, with ultrasound guidance if necessary. A round-tipped spring guide wire is then advanced through the lumen of the trocar, and the trocar is withdrawn. After enlarging the cutaneous puncture with a scalpel, a blunt dilator can now be passed over the guide wire into the cavity or vessel. Then, the tip of a catheter is threaded into the vessel using the spring guide wire. After advancing the catheter into final dwelling position, the spring guide wire is withdrawn.

The endovascular tools are sterile and due to their flexibility and length it is a problem to hold them in one place without making them non-sterile ("they have their own life"). They fall or become non-sterile often. They are hard to handle by one person and hold in an appropriate way. Their cost is very high and a loss of such tools in a procedure has economic implications. Due to their flexibility and length, the operator needs more assistants and it is sometime challenging to handle the tools. Often two to three persons are needed to hold a long catheter/wire.

Most of the labs/angio suites/operation rooms put those tools on a simple table when they are not in use. This obviously does not solve the problem. Some nurses put the wires back to their magazine (time consuming and non practical solution).

There is a real need to have a solution in a form of some placement tool to keep those instruments in their place i.e. that may free some hands (nurse/assistant) and may avoid the falling of a tool or its becoming non-sterile. It is of highest importance in acute procedures. Presently, there are no such placement tools.

US 4,476,860 discloses surgical drapes having a plurality of pockets facing one another for retaining surgical instruments. US 5,010,899 discloses a surgical drape have a plurality of loops for maintaining medical devices during the procedure.US 5,464,025 discloses a self-contained surgical tubing management system where all of the required surgical hand pieces and power and fluid tubing, cables and connections are incorporated in a single disposable package. The system includes a sterile sheet that covers at least a portion of the patient's body, and has pockets and fasteners that contain and position the instruments, so that they are easily accessible during the operation process. None of these meet existing needs in a satisfactory manner.

Further attention is drawn to US3503391, disclosing a non-woven surgical shield or cover member provided with adhesive anchoring means, multiple compartment instrument holding pocket members, supply carrying pockets, waterproofing, special construction of the surgical opening, and a rough surfaced reinforcing member near but spaced from surgical opening.

In addition, in US5944014 there is presented a collection tray for use in pelvic procedures includes a single piece molded plastic tray having a planar portion, a basin portion, and a live hinge there between. The basin portion is preferably provided with a central drain which includes a sponge barrier. The basin portion is also provided with a shelf having one or more slots for holding an irrigation fluid tube so that irrigation fluid may be easily directed toward the site of the procedure.

### SUMMARY OF THE INVENTION

The main object of the present invention is to provide a surgical instrument placement tool for holding tools for angiographic procedures in the fields of invasive radiology, vascular surgery and cardiology.

That object is achieved in accordance with the present invention in that the placement tool comprises:
- a surgical drape for covering a patient of average size from at least the groin down to the region of the feet of the patient when the patient is lying with spread legs;
- a horizontal pouch member attached to a foot end of the surgical drape at a location horizontally to a lower portion of the spread legs of the patient, said pouch member having a vertical double curvature bottom surface and an open side facing vertically upward; and preferably
- a plurality of tool holders attached to an upwardly exposed surface of the surgical drape and extending in a direction across a space between the spread legs of the patient.

Thanks to the vertical double curvature bottom surface, especially in combination with the plurality of tool holders such a placement tool will keep the often very long and springy endovascular instruments in their place. This will free some hands (nurse/assistant) and will avoid the falling of a tool or its becoming non-sterile.

To facilitate the use of long springy instruments, the pouch member may project horizontally beyond the feet of the lying average sized patient. A suitable size of the projection of the pouch member is on the order of half a meter, and then the double curvature bottom surface has a horizontal diameter on the order of half a meter.

Preferably, an arcuate member is located in the pouch member for maintaining the shape of the double curvature bottom surface.

The arcuate member suitably has two end portions extending horizontally between the spread legs of the lying average size patient, and the surgical drape has two pockets for receiving and holding at least the very ends of said end portions. By balancing the weight of the end portions against the combined weight of the pouch member and the arcuate central portion of the arcuate member, the pouch member will project horizontally in cantilever fashion from the foot end of the surgical table.

To hold the long springy instruments and other surgical tools safely without preventing them from being moved in their longitudinal direction, the tool holders suitably are substantially comb-shaped but have comb teeth that are thicker at their tips than at their roots, so that the space between the teeth is smaller at the tips than at the roots.

The tool holders preferably are made of rubber or similar elastic material. As the teeth tips on both sides of the space will be pressed away from each other on insertion of the long springy instruments and other surgical tools in the tool holders, it will be easy to mount the instruments in the tool holders and they will be retained therein. A single-hand grip is sufficient for inserting them into the tool holder and removing them there from.

Since in a majority of procedures at least 7-8 tools are used, the number of spaces between the comb teeth suitably is on the order of ten.

To facilitate the mounting of the long springy instruments and other surgical tools in the tool holders and later handling of them, at least some of the spaces between the teeth of the tool holders may be color coded.

Preferably, the tool holders are attached to the surgical drape by tape having adhesive on both sides.

The upwardly exposed interior surface of the surgical drape may be marked with recommended locations for attaching the tool holders so as to facilitate the attachment at optimum locations.

In addition to said foot end, the surgical drape has an opposed upper end, and to fix the placement tool to the patient, the upper end has one side of a piece of tape having adhesive on both sides adhered thereto, the other side carrying a removable protective film for adhering the placement tool to the patient.

To securely hold the placement tool to the patient, the length of the tape at the upper end of the surgical drape may be sufficient to extend a width across the patient of 30-50 cm (12-20 in.). If desired, the upper end of the surgical drape may have a wing extending toward a nave of the patient.

To be soft, tight and strong, the surgical drape is made from a laminate having a middle layer of plastic film covered by fibrous surface layers.

Also the pouch member should be soft, tight and strong and is made from a laminate having a middle layer of plastic film covered by fibrous surface layers. The plastic film preferably is reinforced by embedded fibers to facilitate the maintaining of the shape of the pouch member. Alternatively or additionally, one or more stiffening members may be provided in or bonded to the laminate pouch member to assist in maintaining its desired shape. With plastics in the pouch member, it can be attached to the surgical drape be welding.

Another object of the present invention is to provide tool holder for holding tools for angiographic procedures in the fields of invasive radiology, vascular surgery and cardiology in case a surgical team operates at a table of extra length.

That object is achieved in accordance with the present invention in that the tool holder is substantially comb-shaped but has comb teeth that are thicker at their tips than at their roots, so that the space between the teeth is smaller at the tips than at the roots, and that the tool holder has a back side having one side of a piece of tape having adhesive on both sides adhered thereto, the other side carrying a removable protective film for adhering the tool holder to any useful surface, such as an ordinary sterile drape.

Thanks to the use of at least one tool holder, it will be easier to maintain the often very long and springy endovascular instruments in their place. This will free some hands (nurse/assistant) and will assist in avoiding the falling of a tool or its becoming non-sterile.

As disclosed above, the tool holder preferably is made of an elastic material, the number of spaces between the comb teeth is on the order of ten, and suitably at least some of the spaces between the teeth of the tool holders are color coded.

If the tool holders are acquired separately and not as included in the surgical instrument placement tool of the invention, a pack containing four tool holders appears to satisfy most requirements. Four tool holders can easily be positioned so as to permit the long springy instruments to form a loop, in which they are movable in their longitudinal direction but yet maintained comparatively safe in the tool holders.

### BRIEF DESCRIPTION OF THE DRAWINGS

In the following, the invention will be described in more detail with reference to preferred embodiments and the appended drawings.
- Fig. 1: is a schematic perspective view of a preferred embodiment of the surgical instrument placement tool of the present invention positioned on a patient lying on a surgical table, an endovascular instrument being held in the placement tool.
- Fig. 2: is a schematic perspective view of a horizontal pouch member included in the placement tool of Fig. 1 and having a vertical double curvature bottom surface and an open side facing vertically upward and having reinforcements to facilitate the maintaining of its shape.

- Fig. 3: is a schematic perspective view of a generally comb-shaped tool holder included in the placement tool of Fig. 1 and having a spring guide wire passing through a space between two teeth.
- Fig. 4: is a schematic plan view of a lower portion of a patient lying on a surgical table and having the surgical instrument placement tool of Fig. 1 covering his legs and the lower part of his abdomen.

### MODE(S) FOR CARRYING OUT THE INVENTION

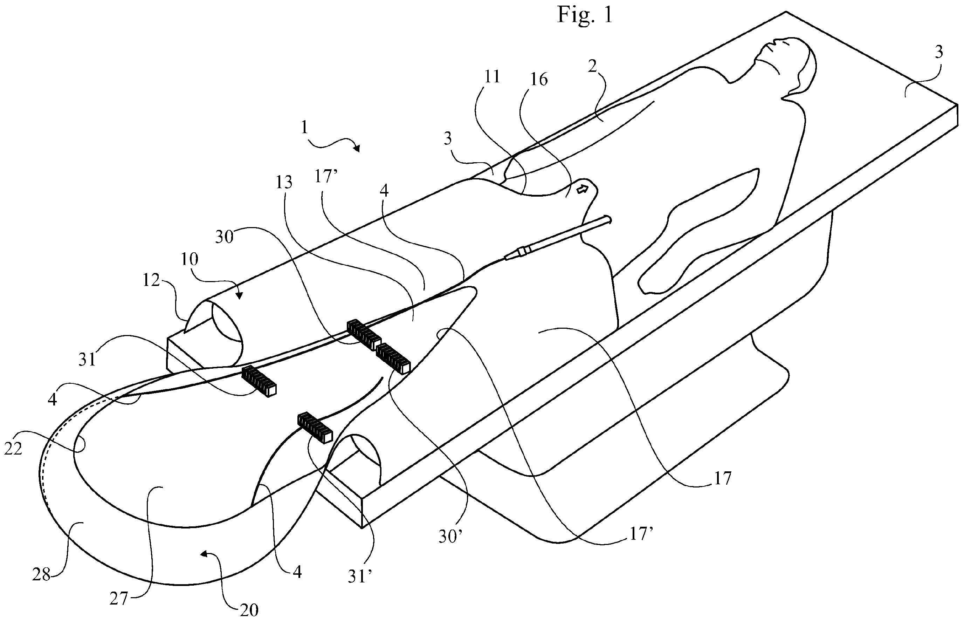

Fig. 1 is a schematic perspective view of a preferred embodiment of the surgical instrument placement tool 1 of the present invention positioned on a patient 2 lying on his back with spread straight legs on a surgical table 3. Although the upper portion of the patient is shown uncovered, it would in real life normally be essentially covered by sterile drapes in accordance with general hospital practice. An endovascular instrument 4 is shown held in the placement tool 1, e.g. to be and is introduced percutaneously or through the skin, into a large blood vessel of the patient 2. Typically the blood vessel chosen is the femoral artery or vein found near the groin. Access to the femoral artery for example, is required for coronary, carotid, and cerebral angiographic procedures. When the instrument 4 is a catheter, it can be used for inserting an intravascular balloon, stent or coil. Stents and coils are composed of fine wire materials such as platinum and can be expanded into a predetermined shape once they are guided into place. Of course, even though the shown instrument is an endovascular one, the present invention is useful for holding also other tools for angiographic procedures in the fields of invasive radiology, vascular surgery and cardiology.

The placement tool 1 comprises:
- a surgical drape 10 for covering a patient 2 of average size from at least the groin down to the region of the feet of the patient when the patient is lying with spread legs to provide a placement surface 13;
- a pouch member 20, having a generally horizontal, upwardly exposed, bottom surface 27, which pouch member 20 is attached to a foot end 12 of the surgical drape 10 to extend to a lower portion of the spread legs of the patient 2, said pouch member 20 having a vertical wall portion 28 providing a double curvature bottom surface 21 and an edge 22 that provides for exposing a portion of said upwardly exposed bottom surface 27 of the pouch member 20; and preferably
- a plurality of tool holders 30, 30', 31, 31' attached to an upwardly exposed surface 13 of the surgical drape 10 and extending in a direction across a space between the spread legs of the patient 2.

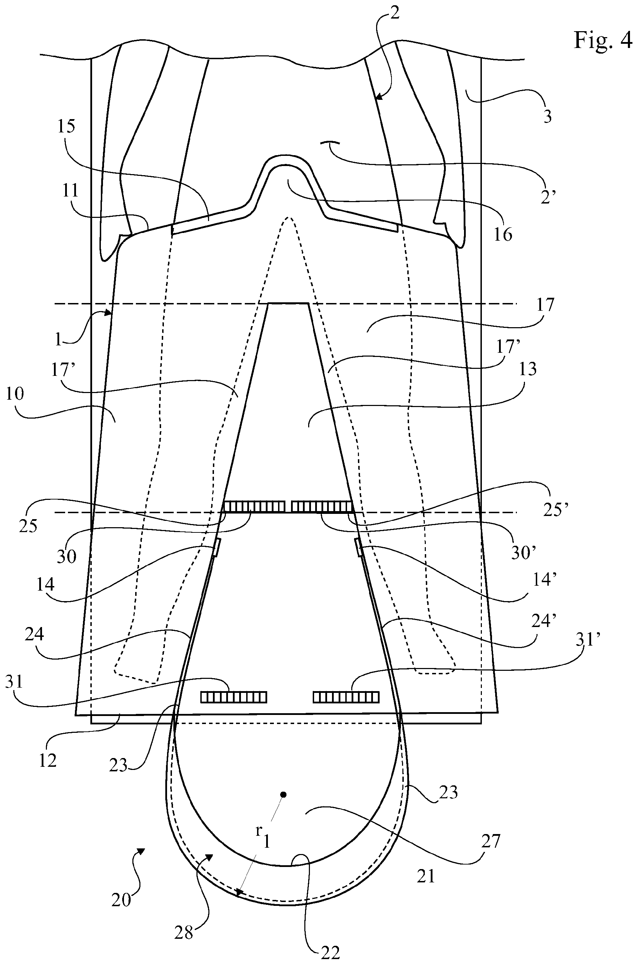

As shown in Figs. 1 and 4, the pouch member 20 projects horizontally beyond the feet of the lying average size patient 2 a distance, which generally is on the order of half a meter (preferably in the range of 03-0,8 m), and the double curvature bottom surface 21 has a horizontal diameter (i.e. two times r₁, see fig 4) that generally is of about the same length. The pouch 20 may be produced as a separate unit that is attached to the drape 10 in any appropriate manner, e.g. gluing and/or welding (plastic).

The generally horizontal upwardly exposed bottom surface 27 of the pouch member 20 extends substantially in the same plane as the plane of the upwardly exposed, generally horizontal, lower surface 13 of the surgical drape 10. The back of the lower surface 13 of the surgical drape 10 normally rests directly on the upper surface of the surgical table 3, i.e. positioning the exposed surfaces 13, 27 generally in level with the top face of the surgical table 3. In the embodiment shown the lower surface 13 of the surgical drape 10 is integrated with leg covering portions 17, which form upwardly extending, inner side walls 17' that partly surrounds the lower surface 13, to form a practical space for placement of tools. In a similar manner the pouch member 20 also presents a space for placement of tools (if desired) within that area thereof, that is exposed within that is positioned centrally in relation to the edge 22 of the wall member 28. Thanks to forming the wall member 28 presenting a vertical double curvature bottom surface 21 the sometimes very long and springy surgical instruments are hindered from escaping out away from the working space, i.e. the space surrounded by the leg portions 17 of the drape 10 and the wall 28 of the pouch. This will free some hands (nurse/assistant) and will avoid the falling of a tool or its becoming non-sterile. Furthermore the walls 17',28 may preferably be arranged such that they totally surround the placement areas 13,27, whereby liquids are hindered from escaping.

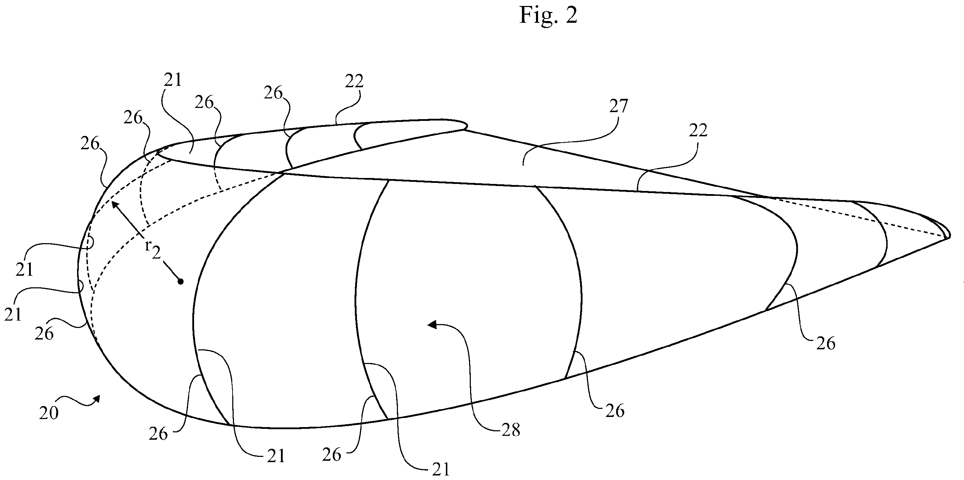

The double curvature bottom surface 21, has a first curvature with a relatively large radius r₁, that forms a U-shape of the inner surface 21 of the wall 28 (and since all parts of the wall 28 have substantially the same thickness also the outer periphery is U-shaped, as seen from above in fig. 4), to allow a smooth continuous bend long springy instruments. Further it has a second curvature with a relatively small radius r₂, along the inner surface 21 in a transversal direction, that also present a U-shape (and an outer U-shaped periphery) seen from the side (see fig 2) to retain the long, springy instruments within the channel that is formed by the cross sectional design of the inner surface 21 of the wall 28. r₂ preferably is within the range 20- 100 mm, but it is evident for the skilled person that a big variety of cross sectional shapes of the surface may be used, to obtain the desired retaining function, i.e. a shape that deviates from a smooth U-shape, e.g. in an extreme embodiment having a U with rectangular corners.

In Fig. 4, an arcuate member 23 is shown located in the pouch member 20 for maintaining the shape of the double curvature bottom surface 21 at least in a generally horizontal plane. The arcuate member 23 may be of any suitable shape and material, e.g. a plastic bar or tube. In the embodiment shown in Fig. 4, the arcuate member 23 has two end portions 24, 24' extending horizontally between the spread legs of the lying average size patient 2. At least the very ends of said end portions 24, 24' are received and held in e.g. two pockets 14, 14' provided on the surgical drape 10. In the embodiment of Fig. 4, these pockets are situated generally on level with and close to and between the thickest portions of the calves of the spread legs of the lying average size patient 2 i.e. in the transition zone between the placement surface 13 and the inner wall member 17. By balancing the weight of the end portions 24, 24' against the combined weight of the pouch member 20 and the arcuate central portion of the arcuate member 23, the pouch member 20 will project horizontally in cantilever fashion from the foot end of the surgical table 3. Further, in Fig. 4 reference numeral 2' designates a possible location for the introduction of the endovascular instrument 4 into a large blood vessel such as the femoral artery or vein of the patient 2.

In the embodiment shown in Figs. 1 and 4, four tool holders 30-31' are used but, if desired, it is possible to use a larger or smaller number of tool holders. The four tool holders 30-31' shown are attached to the surgical drape 10 with a first pair 30, 30' forming a straight line just inside of the pockets 14, 14', and the tool holders of the second pair 31, 31' are located spaced from each other and forming a parallel line just inside the foot end of the surgical table 3, where the pouch member 20 is attached to the surgical drape 10. Of course, the four tool holders 30-31' may be attached to the surgical drape 10 at other locations, if desired.

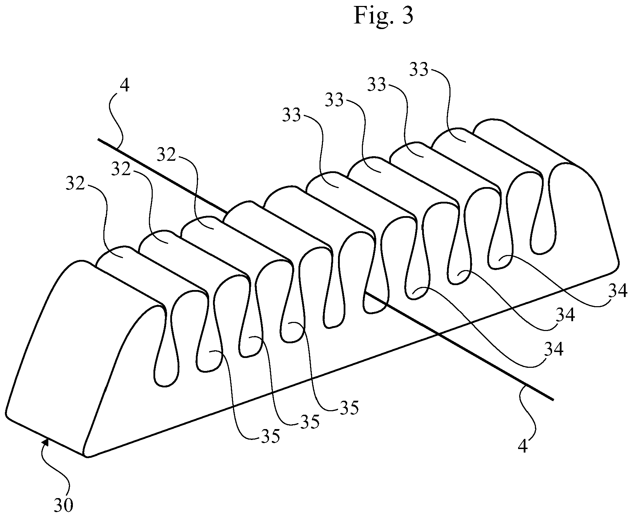

In the preferred embodiment shown in Fig. 3, the tool holders 30-31' are substantially comb-shaped but have comb teeth 32 that are thicker at their tips 33 than at their roots 34, so that the space 35 between the teeth 32 is smaller at the tips 33 than at the roots 34. Thereby, the long springy instruments 4 and other surgical tools can be safely held without being preventing from moving in their longitudinal direction.

The tool holders 30-31' preferably are made of rubber or similar elastic material, more preferred of a porous kind which will decrease weight and cost. As the tooth tips 33 on both sides of the space 35 will be pressed away from each other on insertion of the long springy instruments 4 and other surgical tools in the tool holders 30-31', it will be easy to mount the instruments 4 in the tool holders 30-31' and they will be retained therein. A single-hand grip is sufficient for inserting them into the tool holder 30-31' and removing them there from.

In some procedures, 10-20 tools and more are used, but since in a majority of procedures at least 7-8 tools are used, the number of spaces 35 between the comb teeth 32 suitably is on the order of ten.

To facilitate the mounting of the long springy instruments 4 and other surgical tools in the tool holders 30-31' and the later handling of them, at least some of the spaces 35 between the teeth of the tool holders 30-31' may be color coded (not shown).

Preferably, the tool holders 30-31' are attached to the surgical drape 10 by tape (not shown) having adhesive on both sides, but if desired it is, of course, possible to use some other attachment method.

The upwardly exposed interior surface of the surgical drape 4 may be marked with recommended locations for attaching the tool holders 30-31' so as to facilitate the attachment at optimum locations. In the drawings, the optimum locations are not shown separately, but in most cases, the positions shown in Figs. 1 and 4 are optimum locations.

In addition to said foot end 12, the surgical drape 10 has an opposed upper end 11. To fix the placement tool 1 to the patient 2, the upper end 11 has one side of a piece of tape 15 having adhesive on both sides adhered thereto, the other side carrying a removable protective film, not shown, for adhering the placement tool 1 to the patient 2 after removal of the protective film

To securely hold the placement tool 1 to the patient, the length of the tape 15 at the upper end 15 of the surgical drape 10 may be sufficient to extend a width across the patient of 30-50 cm (12-20 in.). If desired, the upper end 11 of the surgical drape 10 may have a wing 16 extending toward a nave of the patient 2. The wing 16 may carry an instruction (not shown), if desired, as to direction when preparing the patient on the surgical table for surgery.

To be soft, tight and strong, the surgical drape 10 preferably is made from a flexible laminate e.g. of a kind that is known per se and used already as surgical drapes, having a middle layer of plastic film covered by fibrous surface layers. As an example, the film may be a liquid-impermeable polyethylene film, the top fibrous surface layer may be of a liquid-absorbent nonwoven material, and the bottom fibrous surface layer facing the patient 2 may be of cellulose wadding or the like to enhance patient comfort by absorbing perspiration and preventing direct contact of the patient's skin with the plastic film. However, to provide the desired functionality many other combinations of layers and materials therein are possible.

Also the pouch member 20 should be soft, tight and strong and is made from a flexible laminate. The laminate used here will not come into contact with the patient's skin, so the bottom layer of cellulose wadding or the like may not be needed. Thus, the laminate used in the pouch member 20 may have a bottom layer of plastic film covered by a fibrous surface layer. As in the surgical drape 10, the film may be a liquid-impermeable polyethylene film, and the top fibrous surface layer may be of a liquid-absorbent nonwoven material or a woven fabric. For many applications it is preferred to use the same laminate in the pouch 20 as in the drape 10, e.g. to produce them more or less as an integrated unit. However, to arrange for maintaining a desired shape of the pouch member 20, the laminate, e.g. by integration into the plastic film may be reinforced by embedded fibers, *e.g.* glass fibers or a woven polyester fabric, to facilitate the maintaining of the shape of the pouch member. Alternatively or additionally, one or more stiffening members 26 may be provided in or bonded to the laminate pouch member 20 to assist in maintaining its desired shape. Furthermore it is foreseen that the desired shape may be created by punching and gluing of the laminate (preferably having some resiliency) to form the desired shape and strength.

In the embodiment shown in Fig. 2, the generally vertical bottom of pouch member 20 is provided with a plurality of generally vertical stiffening members 26. The stiffening members 26 may be located on the exterior plastic film side or on the interior fibrous surface layer side of the pouch member bottom or on both sides. As an example, they may be provided by laying parallel runs of a hardening or solidifying plastic material on the pouch member bottom and maintaining the shape of the bottom until the plastic material has hardened or solidified, preferably thereafter providing a resiliency such that the drape/pouch 10/20 may be kept flat in its packaging and automatically expand to its intended shape when unpacked. If desired, it is of course also possible to lay two or more parallel runs (not shown) crossing each other to create a net structure of stiffening members. Alternatively, a preformed net-shaped stiffening member (not shown) may be attached to the pouch member 20. For the skilled person within the polymeric field it is evident that there exist a large variety of suitable plastic materials for making the parallel runs/stiffening members 26.

If an arcuate member 23 is used and the stiffening members 26 are located on the interior side of the bottom surface 21 of the pouch member 20, the stiffening members 26 may be shaped, in an embodiment not shown, to provide attachment points, for the arcuate member 23. Such attachment points may be formed as simple shallow recesses or as more complicated snap connections. Further it is foreseen that the arcuate member 23 and stiffening members are integrated to form a unit which may provide the desired shape of flexible drapes 10 and pouches 20, upon introduction thereof. Hence in such an embodiment it may be preferred to have the drape/pouch 10/20 disposable, but the arcuate member/stiffening member 23/26 reusable (hence in a material facilitating sterilization)

A preferred laminate for the pouch member 20 is a traditional kind for surgical drapes. However, to provide the desired functionality, *inter alia* sufficient strength and flexibility, many other combinations of layers and materials therein are possible. The plastic film in the pouch member 20 may be useful not only for tightness purposes but makes it possible to attach the pouch member 20 to the surgical drape 10 by welding.

### INDUSTRIAL APPLICABILITY

The most important field of the surgical instrument placement tool, the separate tool holders and the pack thereof in accordance with the present invention is endovascular surgery and invasive radiology but even invasive cardiology. Both peripheral and central vascular procedures benefit from this tool, and both time and money in these procedures will be saved. Endovascular surgery is performed by radiologists, neurologists, neurosurgeons, cardiologists, and vascular surgeons. The field is rapidly growing as its minimally invasive techniques offer an immediate advantage over more traditional, yet highly invasive surgeries.

The invention is not limited to what has been described above but maybe be varied within the scope of the appended claims. For instance, it is evident that the main function of the invention, i.e. the ability to hinder catheters and other long instruments to fall out/on to the floor, is obtained also without any tool holders positioned on to the drape and/or pouch. Furthermore it is evident that the material of the drape and/or pouch may vary within wide frames to fulfill the desired basic functionality and that different properties may be achieved by alternate laminate/s and/or the manner of combining laminates and/or the manner of gluing the laminates and/or the manner of attaching support members, etc. For instance it is evident that by the use of gluing of layers on to each other and by varying the mounting glue and the amount of layers that are glued to each other, varying properties may be achieved, e.g. to adapt to different needs for different kind of applications. Furthermore it is evident that also the tool holders may be made in a big variety of different materials, e.g. depending on need and/or desires and/or different standards, and/or different requirements regarding different possible aspects. For instance, one aspect that, at least in some parts of the world, is getting more and more of attention is the environmental aspect, Hence an evident alternative regarding the invention is to use materials that are biologically decomposable, as are many fiber products and indeed also polymers of today. In a preferred embodiment all of the parts of the invention should be disposable and in such a case it would of course be an advantage to also have all parts produced in such environmental friendly materials. Furthermore it is evident that to achieve a desired strength to hold the pouch in an essential horizontal position many other means may be used than the above described support device 23, e.g. a flat relatively thick portions in the bottom layer of the drape and pouch. Moreover, it is evident that the leg portions 17 of the drape, do not have to reach all over the legs (as shown in the figures), but that in some applications/situations it is sufficient to merely provide the inner portions 17' (e.g. combined with tape to keep them in place).

## Claims

1. A surgical instrument placement tool (1) for holding tools for angiographic procedures in the fields of invasive radiology, vascular surgery and cardiology, comprising:
- a surgical drape (10) arranged to cover a patient (2) from at least the groin down to the region of the feet, providing a placement surface (13) between the legs of the patient when the patient is lying with spread legs,
- a pouch member (20), having a horizontal, upwardly exposed, bottom surface (27), the pouch member (20) being attached to a foot end (12) of the surgical drape (10) to extend below the spread legs of the patient (2) and projecting horizontally beyond the feet of the lying patient (2),
**characterized in that**:
- the pouch member (20) comprises a vertical wall portion (28) providing a double curvature bottom surface (21) and an edge (22) that provides for exposing a portion of said upwardly exposed bottom surface (27) of the pouch member (20),
- the double curvature wall (28) has a first curvature having a horizontal radius (r₁) that is substantially larger than a second curvature having a vertical radius (r₂), and
- the first and the second curvature each form a U-shape at the double curvature bottom surface (21) of the vertical wall portion (28).

2. The placement tool as claimed in claim 1, wherein at least one tool holder (30-31') is, preferably a plurality of tool holders (30-31') are, attached to said placement surface (13) of the surgical drape (10).

3. The placement tool as claimed in any one of claims 1-2, wherein an arcuate member (23) is located in the pouch member (20) for maintaining the shape of the double curvature bottom surface (21).

4. The placement tool as claimed in claim 3, wherein the arcuate member (23) has two end portions (24, 24') extending horizontally between the spread legs of the lying patient (2), the surgical drape (10) having members (14, 14') for receiving and holding at least the very ends of said end portions (24, 24').

5. The placement tool as claimed in any one of claims 2-4, wherein said tool holder (30-31') is substantially comb-shaped but has comb teeth (32) that are thicker at their tips (33) than at their roots (34), so that the space (35) between the teeth (32) is smaller at the tips (33) than at the roots (34).

6. The placement tool as claimed in claim 5, wherein the tool holder (30-31') is made of an elastic material.

7. The placement tool as claimed in claim 5 or 6, wherein the number of spaces (35) between the comb teeth (32) is on the order of 5-15.

8. The placement tool as claimed in any one of claims 5-7, wherein at least some of the spaces (35) between the teeth (32) of the tool holders (30-31') are color coded.

9. The placement tool as claimed in any one of claims 5-8, wherein the tool holders (30-31') are attached to the surgical drape (10) by tape having adhesive on both sides.

10. The placement tool as claimed in any one of claims 5-9, wherein the upwardly exposed surface (13, of the surgical drape (10), and or pouch (27), is marked with recommended locations for attaching the tool holders (30-31').

11. The placement tool as claimed in any one of claims 1-10, wherein the surgical drape (10) in addition to said foot end (12) has an opposed upper end (11), and the upper end (11) has an adhesive device (15), preferably carrying a removable protective film, for adhering the placement tool to the patient (2).

12. The placement tool as claimed in claim 11, wherein the adhesive device (15) at the upper end (11) of the surgical drape (10) is sufficient to extend a width across the patient (2).

13. The placement tool as claimed in claims 11 or 12, wherein the upper end (11) of the surgical drape (10) has a wing (16) extending toward a nave of the patient (2).

14. The placement tool as claimed in any one of claims 1-13, wherein the surgical drape (10) and/or the pouch member (20) is made from a laminate preferably having a middle layer of plastic film covered by fibrous surface layers.

15. The placement tool as claimed in claim 14, wherein one or more stiffening members (26) is arranged at the pouch member (20) to assist in maintaining its desired shape.

16. The placement tool as claimed in any of claims 1-15, further comprising a tool holder for holding tools for angiographic procedures in the fields of invasive radiology, vascular surgery and cardiology in case a surgical team operates at a table of extra length, wherein the tool holder (30) is substantially comb-shaped but has comb teeth (32) that are thicker at their tips (33) than at their roots (34), so that the spaces (35) between the teeth (32) are smaller at the tips (33) than at the roots (34), and that the tool holder (30) has a back side having an adhesive arranged thereto, for adhering the tool holder to the drape (10).

17. The placement tool as claimed in claim 16, wherein the tool holder is made of an elastic material, and preferably that at least some of the spaces (35) between the teeth (32) of the tool holder (30) are color coded.

18. The placement tool as claimed in claim 16 or 17, wherein the number of spaces (35) between the comb teeth (32) is on the order of 5-15, preferably 10.

## Patentansprüche

1. Platzierungswerkzeug (1) für chirurgische Instrumente zum Halten von Werkzeugen für angiographische Verfahren in den Fachbereichen invasive Radiologie, Gefäßchirurgie und Kardiologie, umfassend:
- eine chirurgische Abdeckung (10), die angeordnet ist, um einen Patienten (2) von mindestens der Leiste bis in den Bereich der Füße abzudecken, wobei sie eine Ablagefläche (13) zwischen den Beinen des Patienten bereitstellt, wenn der Patient mit gespreizten Beinen liegt,
- ein Beutelelement (20), das eine horizontale, nach oben freiliegende Bodenfläche (27) aufweist, wobei das Beutelelement (20) an einem Fußende (12) der chirurgischen Abdeckung (10) befestigt ist, um sich unterhalb der gespreizten Beine des Patienten (2) zu erstrecken und horizontal über die Füße des liegenden Patienten (2) hinaus vorzustehen,
**dadurch gekennzeichnet, dass**:
- das Beutelelement (20) einen vertikalen Wandabschnitt (28) umfasst, der eine doppelt gekrümmte Bodenfläche (21) und eine Kante (22) bereitstellt, die das Freilegen eines Abschnitts der nach oben freiliegenden Bodenfläche (27) des Beutelelements (20) ermöglicht,
- die doppelt gekrümmte Wand (28) eine erste Krümmung mit einem horizontalen Radius (r₁) aufweist, der wesentlich größer ist als eine zweite Krümmung mit einem vertikalen Radius (r₂), und
- die erste und die zweite Krümmung jeweils eine U-Form an der doppelt gekrümmten Bodenfläche (21) des vertikalen Wandabschnitts (28) bilden.

2. Platzierungswerkzeug nach Anspruch 1, wobei mindestens eine Werkzeughalterung (30 - 31'), vorzugsweise eine Vielzahl von Werkzeughalterungen (30 - 31'), an der Platzierungsfläche (13) der chirurgischen Abdeckung (10) befestigt ist.

3. Platzierungswerkzeug nach einem der Ansprüche 1 bis 2, wobei ein bogenförmiges Element (23) in dem Beutelelement (20) angeordnet ist, um die Form der doppelt gekrümmten Bodenfläche (21) aufrechtzuerhalten.

4. Platzierungswerkzeug nach Anspruch 3, wobei das bogenförmige Element (23) zwei Endabschnitte (24, 24') aufweist, die sich horizontal zwischen den gespreizten Beinen des liegenden Patienten (2) erstrecken, wobei die chirurgische Abdeckung (10) Elemente (14, 14') zum Aufnehmen und Halten von mindestens den äußersten Enden der Endabschnitte (24, 24') aufweist.

5. Platzierungswerkzeug nach einem der Ansprüche 2 bis 4, wobei die Werkzeughalterung (30 - 31') im Wesentlichen kammartig ist, wobei sie jedoch Kammzinken (32) aufweist, die an ihren Spitzen (33) dicker sind als an ihren Ansätzen (34), sodass der Raum (35) zwischen den Zinken (32) an den Spitzen (33) kleiner ist als an den Ansätzen (34).

6. Platzierungswerkzeug nach Anspruch 5, wobei die Werkzeughalterung (30 - 31') aus einem elastischen Material hergestellt ist.

7. Platzierungswerkzeug nach Anspruch 5 oder 6, wobei die Anzahl der Zwischenräume (35) zwischen den Kammzinken (32) in der Größenordnung von 5 bis 15 liegt.

8. Platzierungswerkzeug nach einem der Ansprüche 5 bis 7, wobei mindestens ein Teil der Zwischenräume (35) zwischen den Zinken (32) der Werkzeughalterungen (30 - 31') farbcodiert ist.

9. Platzierungswerkzeug nach einem der Ansprüche 5 bis 8, wobei die Werkzeughalterungen (30 - 31') mit einem Band, das auf beiden Seiten Klebstoff aufweist, an der chirurgischen Abdeckung (10) befestigt sind.

10. Platzierungswerkzeug nach einem der Ansprüche 5 bis 9, wobei die nach oben freiliegende Oberfläche (13) der chirurgischen Abdeckung (10) und/oder des Beutels (27) mit empfohlenen Positionen für die Befestigung der Werkzeughalterungen (30 - 31') gekennzeichnet ist.

11. Platzierungswerkzeug nach einem der Ansprüche 1 bis 10, wobei die chirurgische Abdeckung (10) zusätzlich zu dem Fußende (12) ein gegenüberliegendes oberes Ende (11) aufweist und das obere Ende (11) eine Klebevorrichtung (15), die vorzugsweise eine entfernbare Schutzfolie trägt, zum Anbringen des Platzierungswerkzeugs an dem Patienten (2) aufweist.

12. Platzierungswerkzeug nach Anspruch 11, wobei die Klebevorrichtung (15) an dem oberen Ende (11) der chirurgischen Abdeckung (10) ausreichend ist, um sich eine Breite über den Patienten (2) zu erstrecken.

13. Platzierungswerkzeug nach Anspruch 11 oder 12, wobei das obere Ende (11) der chirurgischen Abdeckung (10) einen Flügel (16) aufweist, der sich zu einem Nabel des Patienten (2) hin erstreckt.

14. Platzierungswerkzeug nach einem der Ansprüche 1 bis 13, wobei die chirurgische Abdeckung (10) und/oder das Beutelelement (20) aus einem Verbundstoff hergestellt ist, der vorzugsweise eine mittlere Schicht aus Kunststofffolie aufweist, die von faserigen Oberflächenschichten bedeckt ist.

15. Platzierungswerkzeug nach Anspruch 14, wobei ein oder mehrere Versteifungselemente (26) an dem Beutelelement (20) angeordnet sind, um das Aufrechterhalten seiner gewünschten Form zu unterstützen.

16. Platzierungswerkzeug nach einem der Ansprüche 1 bis 15, ferner umfassend eine Werkzeughalterung zum Halten von Werkzeugen für angiographische Verfahren in den Fachbereichen invasive Radiologie, Gefäßchirurgie und Kardiologie, für den Fall, dass ein Chirurgenteam an einem Tisch mit Extralänge operiert, wobei die Werkzeughalterung (30) im Wesentlichen kammartig ist, jedoch Kammzinken (32) aufweist, die an ihren Spitzen (33) dicker sind als an ihren Ansätzen (34), sodass die Zwischenräume (35) zwischen den Zinken (32) an den Spitzen (33) kleiner sind als an den Ansätzen (34), und dass die Werkzeughalterung (30) eine Rückseite mit einem daran angeordneten Klebstoff aufweist, um die Werkzeughalterung an der Abdeckung (10) zu befestigen.

17. Platzierungswerkzeug nach Anspruch 16, wobei die Werkzeughalterung aus einem elastischen Material hergestellt ist, und bevorzugt ist, dass mindestens ein Teil der Zwischenräume (35) zwischen den Zinken (32) der Werkzeughalterung (30) farbcodiert ist.

18. Platzierungswerkzeug nach Anspruch 16 oder 17, wobei die Anzahl der Zwischenräume (35) zwischen den Kammzinken (32) in der Größenordnung von 5 bis 15, vorzugsweise bei 10 liegt.

## Revendications

1. Dispositif de mise en place d'instrument chirurgical (1) servant à retenir des outils dans des procédures angiographiques dans les domaines de la radiologie invasive, de la chirurgie vasculaire et de la cardiologie, comprenant :
- un champ opératoire (10) placé pour recouvrir un patient (2) au moins depuis l'aine jusqu'à la région des pieds, fournissant une surface de mise en place (13) entre les jambes du patient du patient lorsque le patient est couché avec les jambes écartées;
- un élément formant poche (20), présentant une surface inférieure horizontale exposée vers le haut (27), l'élément formant poche (20) étant attaché à une extrémité pied (12) du champ opératoire (10) pour s'étendre sous les jambes écartées du patient (2) et se projetant horizontalement au-delà des pieds du patient allongé (2),
**caractérisé en ce que** :
- l'élément formant poche (20) comporte une partie paroi verticale (28) fournissant une surface inférieure à double courbure (21) et un bord (22) qui permet d'exposer une partie de ladite surface inférieure exposée vers le haut (27) de l'élément formant poche (20),
- la paroi à double courbure (28) présente une première courbure ayant un rayon horizontal (r₁) qui est sensiblement plus grande qu'une seconde courbure ayant un rayon vertical (r₂), et
- la première et la deuxième courbure forment chacune une forme de U au niveau de la surface inférieure à double courbure (21) de la partie paroi verticale (28).

2. Dispositif de mise en place selon la revendication 1, dans lequel au moins un porte-outils (30 - 31') est, de préférence une pluralité de porte-outils (30 - 31') sont, fixés sur ladite surface de mise en place (13) du champ opératoire (10).

3. Dispositif de mise en place selon l'une quelconque des revendications 1 et 2, dans lequel un élément arqué (23) se trouve dans l'élément formant poche (20) pour maintenir la forme de la surface inférieure à double courbure (21).

4. Dispositif de mise en place selon la revendication 3, dans lequel l'élément arqué (23) a deux parties d'extrémité (24, 24') s'étendant horizontalement entre les jambes écartées du patient allongé (2), le champ opératoire (10) présentant des éléments (14, 14') pour recevoir et retenir au moins les extrémités desdites parties d'extrémités (24, 24').

5. Dispositif de mise en place selon l'une quelconque des revendications 2 à 4, dans lequel ledit porte-outils (30 - 31') est sensiblement en forme de peigne mais a des dents de peigne (32) qui sont plus épaisses au niveau de leur pointe (33) qu'au niveau de leur racine (34), de telle sorte que l'espace (35) entre les dents (32) est plus petit au niveau des pointes (33) qu'au niveau des racines (34).

6. Dispositif de mise en place selon la revendication 5, dans lequel le porte-outils (30 - 31') est en un matériau élastique.

7. Dispositif de mise en place selon la revendication 5 ou 6, dans lequel le nombre d'espaces (35) entre les dents de peigne (32) est de l'ordre de 5 à 15.

8. Dispositif de mise en place selon l'une quelconque des revendications 5 à 7, dans lequel au moins une partie des espaces (35) entre les dents (32) des porte-outils (30 - 31') ont un code couleur.

9. Dispositif de mise en place selon l'une quelconque des revendications 5 à 8, dans lequel les porte-outils (30 - 31') sont fixés au champ opératoire (10) par un ruban ayant un adhésif sur ses deux côtés.

10. Dispositif de mise en place selon l'une quelconque des revendications 5 à 9, dans lequel la surface exposée vers le haut (13) du champ chirurgical (10) et/ou la poche (27) est marquée d'emplacements recommandés pour fixer les porte-outils (30 - 31').

11. Dispositif de mise en place selon l'une quelconque des revendications 1 à 10, dans lequel le champ opératoire (10) outre ladite extrémité pied (12) a une extrémité supérieure opposée (11) et l'extrémité supérieure (11) a un dispositif adhésif (15), de préférence portant un film de protection amovible, pour faire adhérer le dispositif de mise en place au patient (2).

12. Dispositif de mise en place selon la revendication 11, dans lequel le dispositif adhésif (15) au niveau de l'extrémité supérieure (11) du champ opératoire (10) est suffisant pour étendre une largeur à travers le patient (2).

13. Dispositif de mise en place selon les revendications 11 ou 12, dans lequel l'extrémité supérieure (11) du champ opératoire (10) a une aile s'étendant en direction d'un milieu du patient (2).

14. Dispositif de mise en place selon l'une quelconque des revendications 1 à 13, dans lequel le champ opératoire (10) et/ou l'élément formant poche (20) sont formés en un stratifié ayant de préférence une couche intermédiaire de film plastique couvert par des couches de surface fibreuses.

15. Dispositif de mise en place selon la revendication 14, dans lequel un ou plusieurs éléments raidisseurs (26) sont disposés au niveau de l'élément formant poche (20) pour aider à maintenir sa forme souhaitée.

16. Dispositif de mise en place selon l'une quelconque des revendications 1 à 15, comprenant en outre un porte-outils pour retenir les outils pour des procédures angiographiques dans les domaines de la radiologie invasive, de la chirurgie vasculaire et de la cardiologie dans le cas où une équipe chirurgicale opère au niveau d'une table de plus grande longueur, dans lequel le porte outil (30) est sensiblement en forme de peigne mais a des dents de peigne (32) qui sont plus épaisses au niveau de leur pointe (33) qu'au niveau de leur racine (34), de telle sorte que les espaces (35) entre les dents (32) sont plus petits au niveau des pointes (33) qu'au niveau des racines (34), et le porte-outils (30) a un côté arrière sur lequel est disposé un adhésif, pour faire adhérer le porte-outil au champ (10).

17. Dispositif de mise en place selon la revendication 16, dans lequel le porte-outils est en un matériau élastique, et de préférence au moins une partie des espaces (35) entre les dents (32) du porte-outils (30) ont un code couleur.

18. Dispositif de mise en place selon la revendication 16 ou 17, dans lequel le nombre d'espaces (35) entre les dents de peigne (32) est de l'ordre de 5 à 15, de préférence de 10.
